# EUROPEAN PATENT SPECIFICATION

(11) **EP 0 618 059 B1**
(45) Date of publication and mention of the grant of the patent: **08.10.1997**
(21) Application number: 94200875.6
(22) Date of filing: 30.03.1994
(51) Int. Cl.: B29C 47/04, A61M 25/00

(54) **Method for manufacturing an extrusion profile with variable properties along its length and catheters made in accordance with this method**
Herstellungsverfahren eines Extrusionsprofiles mit in Längerichtung veränderlichen Eigenschaften und nach diesem Verfahren hergestelltes Katheter
Procédé de fabrication d'un profile extrudé avec caractéristiques variables dans la direction longitudinale et cathéter fabriqué suivant ce procédé

(30) Priority: 31.03.1993 NL 9300572
(43) Date of publication of application: 05.10.1994
(73) Proprietor: CORDIS EUROPA N.V., NL-9301 LJ Roden (NL)
(72) Inventor: Bos, Johannes, Miami Lakes FL 33014 (US); Mous, Frans, NL-9203 HP Drachten (NL); Van Muiden, Johannes Gerardus Maria, NL-9301 WK Roden (NL)
(74) Representative: 't Jong, Bastiaan Jacobus

(56) References cited:
- EP-A- 0 369 383
- EP-A- 0 385 942
- EP-A- 0 448 886
- DE-A- 4 032 869
- GB-A- 2 191 145
- US-A- 3 752 617
- US-A- 3 825 036
- US-A- 4 182 582
- US-A- 4 276 250
- US-A- 4 874 305

## Description

The present application relates to a method for manufacturing a medical device, specifically for internal use, such as a catheter, according to the preamble of claim 1, with variable mechanical properties along the length thereof, such as a varying degree of stiffness.

Such a process for manufacturing a catheter is known from US-A-4276250, where different streams of material are turned on during the extrusion process, so that the extrusion profile is longitudinally made up of sections of varying composition.

The object of the invention is to improve a method of the type as described in US-A-4276250.

According to the invention this can be achieved with the measures described in the characterising part of claim 1. The extrusion profile manufactured in accordance with the method according to the invention in cross section has areas of materials of different composition. Along the length of the profile the overall surface area of each of the different types of material may vary and consequently the properties of the extrusion profile will vary.

Other embodiments of medical devices according to the present invention include stents, tubes for introduction of catheters or the like.

In the European patent application no. EP-A-0448886 catheters are described, which are composed of a number of materials distributed evenly in the circumferential direction the catheter. This publication relates to the materials to be used in manufacture of these catheters, where a method for manufacturing these catheters is not disclosed. Furthermore catheters described in this publication do not have varying mechanical properties in axial direction thereof.

A simple and effective embodiment of the process is achieved with the measure as set out in claim 2.

The process of the invention together with the preferred measure as set out in claim 3 is especially useful for the manufacture of catheters such as catheters used for angiographic purposes. Such catheters should be pliable, ie of limited stiffness at the distal end in order to easily follow the course of a blood vessel. More towards the proximal end such a catheter is preferably stiffer in order to convey the pressure, exerted on the catheter on insertion, to its distal end.

A material which displays a greater stiffness than the other materials used could for instance be a solid material such as a fibre bundle. A fibre bundle can easily be incorporated in the extrusion profile during the extrusion process. One could start the extrusion off with for example the maximum number of streams of solid material in the form of fibre bundles whereby, during the course of the extrusion process, these are cut off gradually so that an extrusion profile is formed which is initially stiff comparatively speaking but more pliable towards the end.

Another embodiment of the method of the invention is characterised in claim 5. The advantage of using streams of molten material is that it is not only easy to turn certain streams off but just as easy to turn these on. In the case of a stream of solid material this is more difficult to achieve. By only using streams of molten material it is possible to produce within one length of extrusion profile a number of sections of which the properties alter in the same manner.

The invention relates to and also provides catheters manufactured by a method in accordance with the invention. These catheters according to claim 6 are characterised in that at least partly the wall consists of longitudinal bands of material of different composition.

In order to obtain a desired development of properties in a longitudinal direction the measure as set out in claim 7 is preferably employed. When one wants to vary the stiffness, the cross-section of each band containing the stiffer material can for instance be reduced longitudinally while the cross-section of an adjoining band containing less stiff material increases correspondingly.

In stead of causing the total cross-section of the different bands of material to vary while keeping the number of bands constant, one could also vary the number of bands. In the case of a catheter manufactured in accordance with the method of the invention the number of bands made up of a stiffer material could be reduced gradually towards the distal end of the catheter.

The invention will be explained in greater detail in the following description with reference to the attached drawings.

**Fig. 1** shows schematically the manufacturing method of the invention.

**Fig. 2** shows an extrusion profile manufactured by the method of the invention.

**Fig. 3** shows a cross-section at III-III in fig. 2.

**Fig. 4** shows a cross-section at IV-IV in fig. 2.

**Fig. 5** and **6** show two other extrusion profiles manufactured by the method of the invention.

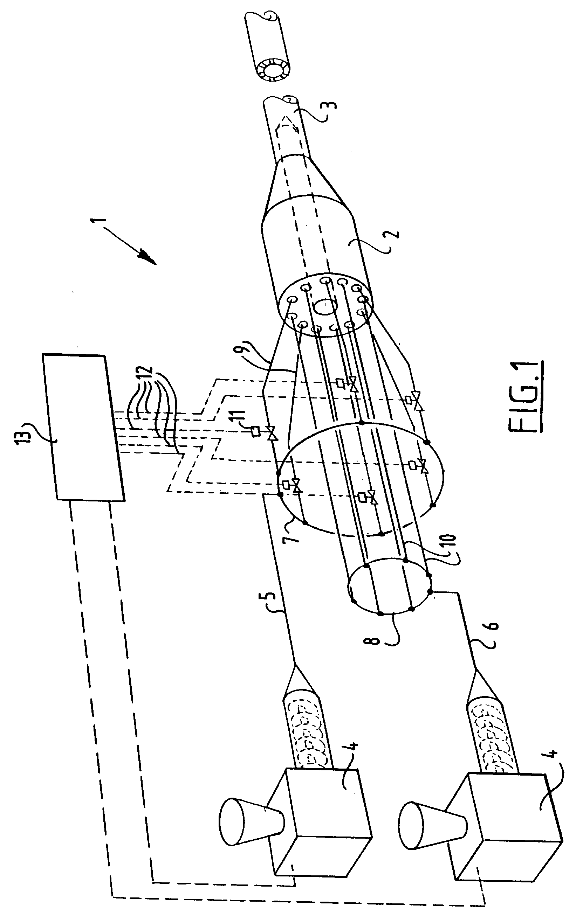

Fig. 1 shows schematically an extrusion device which can be used to carry out the method of the invention. This device 1 comprises a moulding-nozzle 2 in which the extrusion profile 3 is formed. The embodiment of the method as described here, involves the use of streams of material of two different compositions. Each of the materials is placed in an extruder 4 at the correct degree of liquidity and at the right pressure required for extrusion. The material coming from the first extruder 4 is conveyed, through line 5, to a distribution line 7. From this distribution line a number of lines 9 branch off, each of which can convey a stream of material.

The second extruder 4 leads to a line 6, also connected to a distribution line 8 which, in its turn links up with a number of lines 10 conveying separate streams of material.

As fig. 1 shows there are in this embodiment twelve, in the circumferential direction of the tube-like profile 3 distributed streams of material of two different compositions.

The different materials can be incorporated in a pattern of alternate bands in the wall of profile 3.

In each of the lines 9, conveying the streams of material of the first composition, cut-off valves 11 have been arranged. Each of these cut-off valves 11, can be controlled by means of control lines 12 by a control means 13. The control means 13 can open or close the cut-off valves 11 during the extrusion process in a controlled manner and consequently the streams of material conveyed through the corresponding lines 9 can be turned on and off in a similar controlled manner. The control means 13 can be made to control the extruders 4 as well. The opening and closing of the cut-off valves 11 is preferably programmed in a preset cycle. Manual operation is obviously possible as well.

The streams of material conveyed through the lines 9 and 10 flow together in the moulding-nozzle 2. After allowing the combined stream of materials to cool off in the usual manner, the extrusion profile 3 has been formed.

In the embodiment as shown in fig.1 the streams of material through the lines 10 are conveyed continuously and those through the lines 9 can be turned on and off in a controlled manner by the cut-off valves 11.

When the control means 13 closes all cut-off valves 11, the extrusion profile 3 will be made entirely of material conveyed through the lines 10. When a cut-off valve 11 is opened the extrusion profile 3 will contain a longitudinal band made of material coming from the first extruder 4, while the remainder of the cross-section of the profile 3 will be made of material coming from the second extruder 4.

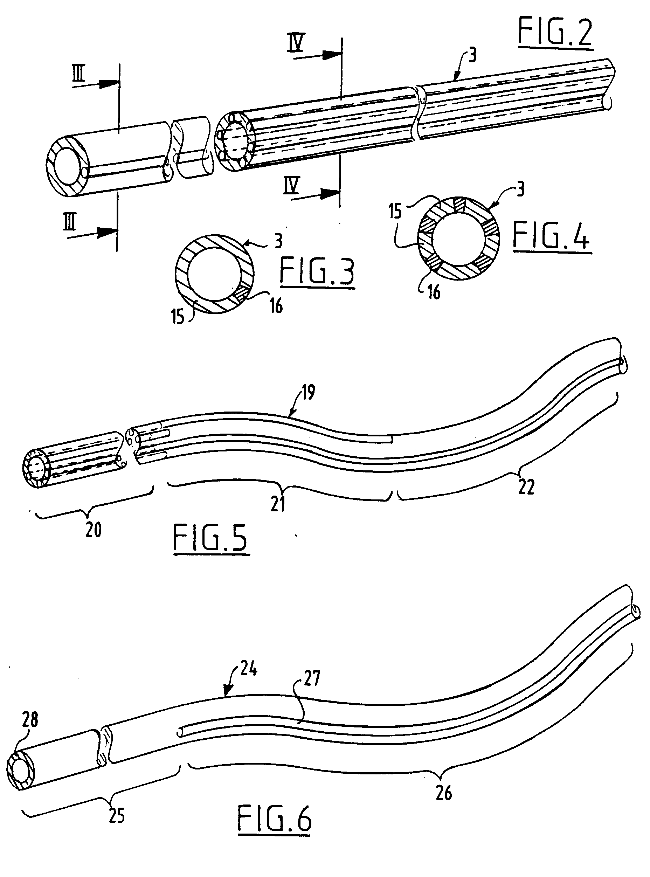

This situation is shown in fig. 3, representing a cross-section at III-III in fig. 2. It can be seen that in the annular cross-section of the catheter 3 there is one band 16 made of material from the first extruder, while the rest of the cross-section consists of material 15 from the second extruder.

When all cut-off valves 11 have been opened, the profile will have a cross-section as shown in fig. 4, in which case material 15 and material 16 alternate in a regular fashion in circumferential direction.

By opening and closing the cut-off valves 11 in a controlled manner the extrusion profile will display varying properties in a linear direction corresponding to the properties of the constituent streams of material. When for instance the material from the first extruder 4 is stiffer when cooled off than the material from the second extruder, the extrusion profile 3 with a cross-section as shown in fig. 3 will be less stiff than a section of the extrusion profile with a cross-section as shown in fig. 4. By varying the number of cut-off valves 11 as desired, the stiffness of the extrusion profile can be made to alter gradually in a linear direction.

Turning the streams of material on and off can be done either abruptly or gradually. When it is done gradually the cross-section of the band made of the stream of material concerned in the extrusion profile will vary accordingly and thus there will be a more gradual transition of the properties of the material.

The section of extrusion profile 19 as shown in fig.5 forms the basic material for a catheter. It is made up of materials of two different compositions. The material of the first composition is, when cooled off, stiffer than that of the second composition. In the first section 20, six bands of the first material have been incorporated in between six bands of the second material. This section 20 is therefore stiff comparatively speaking. The second section 21 constitutes a transitional section and contains two bands of the stiff material while the remainder is made up of the less stiff material. During the extrusion process in this example, four streams of the material of the first composition were turned off at the point of transition from the first section to the second section 21. At the point of transition from the second section 21 to the third section 22 of the material, one of the two remaining streams of material of the first composition was turned off, so that the third section 22 contains only one band of the stiffer material. Consequently the end-section 22 is considerably more pliable than the first section 20. In addition to a property such as the compliance of the material also other properties can be incorporated in the extrusion profile. The basic material 24 for a catheter for instance, comprises a first section 25 made of a material of a first composition while in the second section a band 27 of a different material has been included. Because of this one band 27 the compliance properties of the basic material have been altered. When the basic material 27 has a high tensile stiffness and is made of a fibre bundle for instance, the profile will bend in a particular preferential direction when a longitudinal compressive force is exerted on the end.

Although the above description refers continually to streams of material of two different compositions whereby the streams with a certain composition can be turned on and off at will, the invention is not limited to this. Streams of material with more than two different compositions can be combined and turning the streams of material on and off is not limited to streams of the same composition.

## Claims

1. Method for manufacturing a medical device, specifically for internal use, such as a catheter, formed by a tube-like extrusion profile (3) with an annular cross section and varying mechanical properties in axial direction thereof, comprising:
- simultaneously conveying a number of divided streams (9,10) of material of at least two different compositions to a moulding nozzle (2), wherein the streams of material (9) of the same composition supplied alternating with those (10) of another composition,
- making these streams (9,10) flow together in the moulding nozzle (2) whereby at least one of the streams (9,10) can be turned on and/or off in a controlled manner during the extrusion method, and
- allowing the combined stream of materials to cool off into an extrusion profile,
**characterised in that** - the streams (9,10) of material to be conveyed to the moulding nozzle (2) are divided in the circumferential direction of the profile, and
- in that the streams (9,10) of material of the same composition are distributed evenly in the circumferential direction.

2. Method according to claim 1, wherein the streams (9, 10) which are turned on and/or off in a controlled manner are streams (9, 10) of material of the same composition.

3. Method according to claim 1 or 2, wherein the compositions of the materials differ in such way that the stiffness of those materials is different in the cooled off state.

4. Method according to claim 1, 2 or 3, wherein at least one of the streams (9, 10) is a stream of solid material such as a fibre bundle.

5. Method according to any of the claims 1, 2 or 3, wherein all streams (9, 10) are streams of molten material.

6. Catheter with a tube-like extrusion profile (3) manufactured by the method according to any of the preceding claims containing at least one section the wall of which consists of longitudinal bands (16) of material of different composition.

7. Catheter as claimed in claim 6, wherein the cross-section of at least two adjoining bands (16) varies inversely in longitudinal direction.

8. Catheter as claimed in claim 7, wherein the bands (16) comprise materials of different stiffness and wherein the total cross-section of the bands (16) made of the stiffer material is smaller in the distal end-section of the catheter than in the remaining part.

9. Catheter as claimed in claim 8, wherein the cross-section and/or the number of bands (16) made of the stiffer material decreases from the proximal to the distal end.

## Patentansprüche

1. Verfahren zum Herstellen einer medizinischen Vorrichtung, insbesondere zur Verwendung in der inneren Medizin, wie einem Katheter, die aus einem rohrförmigen Extrusionsprofil (3) mit einem ringförmigen Querschnitt und variierenden mechanischen Eigenschaften in seiner axialen Richtung geformt ist, mit folgenden Schritten:
gleichzeitiges Transportieren einer Anzahl von voneinander getrennten Strömen (9, 10) von Material mit mindestens zwei unterschiedlichen Zusammensetzungen zu einer Formdüse (2) hin, wobei die zugeführten Ströme von Material (9) mit derselben Zusammensetzung mit den Strömen von Material (10) der anderen Zusammensetzung abwechseln,
Zusammenfließenlassen dieser Ströme (9, 10) in der Formdüse (2), wobei mindestens einer der Ströme (9, 10) in einer gesteuerten Weise während des Extrusionsverfahrens freigegeben und/oder unterbrochen werden kann, und
Ermöglichen des kombinierten Stromes von Materialien, sich zu einem Extrusionsprofil abzukühlen,
**dadurch gekennzeichnet**, daß
die zu der Formdüse (2) hin zu transportierenden Ströme (9, 10) von Material mit derselben Zusammensetzung in Umfangsrichtung des Profils voneinander getrennt werden, und
die Ströme (9, 10) von Material mit derselben Zusammensetzung in Umfangsrichtung des Profils gleichmäßig verteilt werden.

2. Verfahren nach Anspruch 1, wobei die Ströme (9, 10), welche in einer gesteuerten Weise freigegeben und/oder unterbrochen werden, Ströme (9, 10) von Material mit derselben Zusammensetzung sind.

3. Verfahren nach Anspruch 1 oder 2, wobei die Zusammensetzungen der Materialien sich in einer solchen Weise voneinander unterscheiden, daß die Steifigkeit dieser Materialien im abgekühlten Zustand unterschiedlich ist.

4. Verfahren nach Anspruch 1, 2 oder 3, wobei mindestens einer der Ströme (9, 10) ein Strom von festem Material, wie einem Faserbündel, ist.

5. Verfahren nach einem der Ansprüche 1, 2 oder 3, wobei alle Ströme (9, 10) Ströme von geschmolzenem Material sind.

6. Katheter mit einem rohrförmigen Extrusionsprofil (3), das nach dem Verfahren gemäß einem der vorhergehenden Ansprüche hergestellt wird, wobei mindestens ein Abschnitt seiner Wand aus Materiallängsstreifen (16) mit anderer Zusammensetzung besteht.

7. Katheter nach Anspruch 6, wobei der Querschnitt mindestens zweier aneinandergrenzender Stränge (16) in Längsrichtung umgekehrt zueinander variiert.

8. Katheter nach Anspruch 7, wobei die Stränge (16) Materialien mit unterschiedlicher Steifigkeit aufweisen, und wobei der Gesamtquerschnitt der Stränge (16), die aus dem steiferen Material hergestellt sind, in dem distalen Endabschnitt des Katheters kleiner als in dem restlichen Bereich ist.

9. Katheter nach Anspruch 8, wobei sich der Querschnitt und/oder die Anzahl der Stränge (16), die aus dem steiferen Material hergestellt sind, von dem proximalen zu dem distalen Ende hin verringert.

## Revendications

1. Procédé de fabrication d'un dispositif médical, spécifiquement pour un usage interne, tel qu'un cathéter, formé par un profilé extrudé (3) semblable à un tube, avec une section transversale annulaire et des propriétés mécaniques variables dans la direction axiale de celui-ci, comprenant les opérations suivantes :
- acheminer simultanément un certain nombre de flux divisés (9, 10) de matériau d'au moins deux compositions différentes en direction d'une buse de moulage (2), les flux de matériau (9) de la même composition étant acheminés en alternance avec ceux (10) d'une autre composition,
- faire en sorte que ces flux (9, 10) s'écoulent ensemble dans la buse de moulage (2), au moins l'un de ces flux (9, 10) pouvant être amené à couler et/ou étant interrompu de façon commandée au cours du procédé d'extrusion, et
- laisser refroidir le flux combiné de matériaux afin de constituer un profilé extrudé,
caractérisé en ce que
- les flux (9, 10) de matériau destinés à être acheminés vers la buse de moulage (2) sont divisés dans la direction circonférentielle du profilé, et
- en ce que les flux (9, 10) de matériau de même composition sont répartis régulièrement dans la direction circonférentielle.

2. Procédé selon la revendication 1, dans lequel les flux (9, 10) qui sont amenés à couler et/ou sont interrompus de façon commandée sont des flux (9, 10) de matériau de la même composition.

3. Procédé selon la revendication 1 ou 2, dans lequel les compositions des matériaux diffèrent de telle sorte que la rigidité de ces matériaux est différente à l'état refroidi.

4. Procédé selon la revendication 1, 2 ou 3, dans lequel au moins l'un des flux (9, 10) est un flux de matériau solide tel qu'un faisceau de fibres.

5. Procédé selon l'une quelconque des revendications 1, 2 ou 3, dans lequel tous les flux (9, 10) sont des flux de matériau fondu.

6. Cathéter avec un profilé extrudé (3) semblable à un tube, fabriqué suivant le procédé selon l'une quelconque des revendications précédentes, comprenant au moins une partie dont la paroi est constituée de bandes longitudinales (16) de matériau de différente composition.

7. Cathéter selon la revendication 6, dans lequel la section transversale d'au moins deux bandes (16) contiguës varie inversement dans la direction longitudinale.

8. Cathéter selon la revendication 7, dans lequel les bandes (16) comprennent des matériaux de rigidité différente, et dans lequel la section transversale totale des bandes (16) constituées en matériau plus rigide est plus réduite dans la région de l'extrémité distale du cathéter que dans la partie restante.

9. Cathéter selon la revendication 8, dans lequel la section transversale et/ou le nombre de bandes (16) constituées en matériau plus rigide diminue de l'extrémité proximale en direction de l'extrémité distale.
